# EUROPÄISCHE PATENTSCHRIFT

(11) **EP 3 150 563 B1**
(45) Veröffentlichungstag und Bekanntmachung des Hinweises auf die Patenterteilung: **22.05.2019**
(21) Anmeldenummer: 15187666.1
(22) Anmeldetag: 30.09.2015
(51) Int. Cl.: C03C 3/097, A61L 27/10, C03C 10/00, C03C 4/00

(54) **LITHIUMSILIKAT-WOLLASTONIT-GLASKERAMIK**
LITHIUM SILICATE WOLLASTONITE GLASS CERAMIC
VITROCERAMIQUE EN SILICATE DE LITHIUM-WOLLASTONITE

(43) Veröffentlichungstag der Anmeldung: 05.04.2017
(73) Patentinhaber: Ivoclar Vivadent AG, 9494 Schaan (LI)
(72) Erfinder: Dittmer, Marc, 6800 Feldkirch (AT); Höland, Wolfram, 9494 Schaan (LI); Rampf, Markus, 8853 Lachen (CH); Schweiger, Marcel, 7000 Chur (CH)
(74) Vertreter: Uexküll & Stolberg

(56) Entgegenhaltungen:
- WO-A1-02/34685
- DE-T2- 69 204 791
- GB-A- 2 224 025
- US-A- 4 515 634
- SAAD M SALMAN ET AL: "Crystallization Behaviour of Some Glasses in the System Li2O - CaO - MgO - SiO2", SPRECHSAAL,, Bd. 118, Nr. 9, 1. September 1985 (1985-09-01), Seiten 782-788, XP001260561,

## Beschreibung

Die Erfindung betrifft Lithiumsilikat-Wollastonit-Glaskeramik, die sich insbesondere zum Einsatz in der Zahnheilkunde, bevorzugt zur Herstellung von dentalen Restaurationen, eignet sowie Vorstufen zur Herstellung der Glaskeramik.

Glaskeramiken mit einer Lithiumsilikat-Kristallphase und deren Verwendung in Dentalprodukten sind aus dem Stand der Technik bekannt. Beispielsweise beschreibt die EP 1 505 041 Lithiumsilikat-Glaskeramiken, die in Form von Lithiummetasilikat-Glaskeramiken mittels CAD/CAM-Verfahren zu den gewünschten Dentalrestaurationen verarbeitet werden, wobei eine anschließende Wärmebehandlung zur Umwandlung der Lithiummetasilikat (Li₂SiO₃)-Phase in Lithiumdisilikat (Li₂Si₂O₅)-Phase und damit zur Ausbildung hochfester Lithiumdisilikat-Glaskeramik führt. Eine maschinelle Bearbeitung der Glaskeramik nach Ausbildung der Lithiumdisilikat-Phase ist insbesondere aufgrund deren hoher Festigkeit zeitaufwendig und mit hohem Werkzeugverschleiß verbunden.

Glaskeramiken, die Wollastonit als Kristallphase enthalten, sind ebenfalls bekannt.

Wollastonit-Glaskeramiken finden vor allem in der Bauindustrie als Fassadenmaterial Anwendung (vgl. Höland, Beall, "Glass-Ceramic Technology", Wiley, USA, 2. Auflage, 2012, S. 114-116) .

Die DD 247 574 beschreibt Glaskeramiken mit Apatit- und Wollastonit als Kristallphasen. Die Glaskeramiken werden zum Knochenersatz verwendet und ihre hohe Bioaktivität führt dazu, dass sie mit dem Knochen im lebenden Organismus einen festen Verbund ausbilden können.

Die DD 262 366 offenbart restaurative Zahnmaterialien aus Glaskeramiken, die Apatit und überdies Wollastonit sowie gegebenenfalls Perowskit, Cristobalit und/oder Sphen als Kristallphasen enthalten.

Die DE 692 04 791 beschreibt Glaskeramiken, die zur Herstellung von Geschirr dienen. Die Glaskeramiken enthalten als vorherrschende Kristallphase Lithiumdisilikat sowie geringe Anteile an Spodumen, Cristobalit und Wollastonit.

Die US 2005/0079226 beschreibt bioaktive Gläser, die in Materialien zum Ersatz von Knochen als Sinterhilfe Verwendung finden. Nach Kristallisation enthalten die Gläser Wollastonit und Diopsid als Kristallphasen.

Aus der US 5,356,436 sind bioaktive Keramiken bekannt, die zum Ersatz von Knochen dienen und bei Kontakt mit Körperflüssigkeiten zum Beispiel Hydroxyapatit auf ihrer Oberfläche ausbilden können. Die Keramiken können z.B. Diopsid, Wollastonit, Alit, Belit, Akermanit, Monticellit, Forsterit, Protoenstatit und Tridymit als Kristallphasen aufweisen.

Die US 5,711,763 beschreibt bioaktive Implantate aus einem metallischen Substrat, in dessen Oberfläche keramische Teilchen eingebettet sind. Die keramischen Materialien können zum Beispiel aus der Gruppe von Diopsid, Wollastonit, Alit, Belit, Akermanit, Monticellit, Forsterit, Protoenstatit und Tridymit stammen. Die US 4,515,634 beschreibt Lithiumdisilikat-Glaskeramiken, die als dentales Restaurationsmaterial eingesetzt werden können.

Die bekannten Glaskeramiken weisen allerdings eine Reihe von Nachteilen auf. Bei ihnen kann in vielen Fällen die Transluzenz nicht über einen breiten Bereich eingestellt werden, wie es für vielseitig einsetzbare Dentalmaterialien wünschenswert ist. Darüber hinaus ist bei ihnen häufig eine einfache maschinelle Bearbeitung nicht möglich. Zudem erweist sich ihre Festigkeit häufig als nicht ausreichend, um ihren Einsatz als restauratives Dentalmaterial zu gestatten.

Der Erfindung liegt die Aufgabe zugrunde, eine Glaskeramik zur Verfügung zu stellen, die über gute optische Eigenschaften, insbesondere eine steuerbare Transluzenz, sowie gute mechanische Eigenschaften verfügt und damit als restauratives Dentalmaterial verwendet werden kann. Die Glaskeramik soll darüber hinaus in einfacher und schneller Weise durch maschinelle Bearbeitung, z.B. mittels CAD/CAM-Verfahren, zu dentalen Restaurationen verarbeitbar sein. Diese einfache Bearbeitung soll insbesondere auch nach möglichst vollständiger Kristallisation der gewünschten Kristallphasen möglich sein.

Diese Aufgabe wird durch die Lithiumsilikat-Wollastonit-Glaskeramik nach den Ansprüchen 1 bis 18 gelöst. Gegenstand der Erfindung sind ebenfalls das Ausgangsglas nach Anspruch 19, das Verfahren nach Anspruch 20 sowie die Verwendung nach den Ansprüchen 21-24.

Die erfindungsgemäße Lithiumsilikat-Wollastonit-Glaskeramik zeichnet sich dadurch aus, dass sie Lithiumsilikat als eine Kristallphase und Wollastonit als eine weitere Kristallphase enthält.

Diese Glaskeramik zeigt überraschenderweise eine vorteilhafte Kombination von für ein restauratives Dentalmaterial wünschenswerten mechanischen und optischen Eigenschaften und sie kann zudem in einer für ein Dentalmaterial vorteilhaften Weise in die gewünschte Form, zum Beispiel einer Dentalrestaurationen, wie einer Krone, gebracht werden.

Die erfindungsgemäße Glaskeramik enthält 55,0 bis 74,0, insbesondere 56,0 bis 73,0 und bevorzugt 60,0 bis 69,0 Gew.-% SiO₂.

Es ist weiter bevorzugt, dass die Glaskeramik 10,0 bis 18,0, insbesondere 11,0 bis 17,0 und bevorzugt 12,0 bis 16,5 Gew.-% Li₂O enthält.

Des Weiteren ist es bevorzugt, dass die Glaskeramik 4,0 bis 17,0, insbesondere 5,0 bis 16,0 und bevorzugt 7,0 bis 15,0 Gew.-% CaO enthält.

Die Glaskeramik enthält 0,5 bis 6,0, insbesondere 0,5 bis 5,0 und bevorzugt 1,0 bis 4,0 Gew.-% Al₂O₃.

Es ist weiter bevorzugt, dass die Glaskeramik 0 bis 5,0, insbesondere 0 bis 4,5 und bevorzugt 0,5 bis 4,0 Gew.-% K₂O enthält.

Weiter enthält die Glaskeramik 1,0 bis 7,0, insbesondere 2,0 bis 6,0 und bevorzugt 3,0 bis 6,0 Gew.-% P₂O₅. P₂O₅ kann insbesondere als Keimbildner für die Bildung von Lithiumsilikat fungieren. Das Vorhandensein eines Keimbildners ist für die Bildung von Lithiumsilikat als Kristallphase jedoch nicht zwingend erforderlich.

Auch ist es bevorzugt, dass die Glaskeramik neben Li₂O und K₂O weiteres Alkalimetalloxid Me^{I}₂O in einer Menge von 0 bis 13,0, bevorzugt 0 bis 12,0 und besonders bevorzugt 0 bis 11,0 Gew.-% enthält, wobei Me^{I}₂O ausgewählt ist aus Na₂O, Rb₂O und/oder Cs₂O.

Besonders bevorzugt enthält die Glaskeramik mindestens eines und insbesondere alle der folgenden weiteren Alkalimetalloxide Me^{I}₂O in den angegebenen Mengen:

| Komponente | Gew.-% |
|---|---|
| Na₂O | 0 bis 7,0, insbesondere 0 bis 6,0 |
| Rb₂O | 0 bis 11,0, insbesondere 0 bis 8,0 |
| Cs₂O | 0 bis 13,0, insbesondere 0 bis 12,0. |

Des Weiteren ist es bevorzugt, dass die Glaskeramik 0 bis 6,0 und bevorzugt 0 bis 5,0 Gew.-% weiteres Oxid zweiwertiger Elemente Me^{II}O enthält, wobei Me^{II}O ausgewählt ist aus MgO, SrO und/oder ZnO.

Besonders bevorzugt enthält die Glaskeramik mindestens eines und insbesondere alle der folgenden Oxide zweiwertiger Elemente Me^{II}O in den angegebenen Mengen:

| Komponente | Gew.-% |
|---|---|
| MgO | 0 bis 3,0, insbesondere 0 bis 2,0 |
| SrO | 0 bis 6,0, insbesondere 0 bis 5,0 |
| ZnO | 0 bis 5,0, insbesondere 0 bis 4,0. |

Es ist weiter eine Glaskeramik bevorzugt, die 0 bis 6,0 und bevorzugt 0 bis 5,0 Gew.-% Oxid dreiwertiger Elemente Me^{III}₂O₃ enthält, wobei Me^{III}₂O₃ ausgewählt ist aus B₂O₃, Y₂O₃, La₂O₃ und/oder Er₂O₃.

Besonders bevorzugt enthält die Glaskeramik mindestens eines und insbesondere alle der folgenden Oxide dreiwertiger Elemente Me^{III}₂O₃ in den angegebenen Mengen:

| Komponente | Gew.-% |
|---|---|
| B₂O₃ | 0 bis 4,0, insbesondere 0 bis 3,5 |
| Y₂O₃ | 0 bis 5,0, insbesondere 0 bis 4,0 |
| La₂O₃ | 0 bis 6,0, insbesondere 0 bis 5,0 |
| Er₂O₃ | 0 bis 2,0, insbesondere 0 bis 1,0. |

Ferner ist eine Glaskeramik bevorzugt, die 0 bis 8,0 und bevorzugt 0 bis 7,0 Gew.-% weiteres Oxid vierwertiger Elemente Me^{IV}O₂ enthält, wobei Me^{IV}O₂ ausgewählt ist aus ZrO₂, GeO₂, CeO₂, TiO₂ und/oder SnO₂.

Besonders bevorzugt enthält die Glaskeramik mindestens eines und insbesondere alle der folgenden weiteren Oxide vierwertiger Elemente Me^{IV}O₂ in den angegebenen Mengen:

| Komponente | Gew.-% |
|---|---|
| ZrO₂ | 0 bis 7,0, insbesondere 0 bis 6,0 |
| GeO₂ | 0 bis 6,0, insbesondere 0 bis 5,0 |
| CeO₂ | 0 bis 3,0, insbesondere 0 bis 2,0 |
| TiO₂ | 0 bis 5,0, insbesondere 0 bis 4,0 |
| SnO₂ | 0 bis 8,0, insbesondere 0 bis 7,0. |

Außerdem ist eine Glaskeramik bevorzugt, die 0 bis 6,0 und bevorzugt 0 bis 5,0 Gew.-% weiteres Oxid fünfwertiger Elemente Me^{V}₂O₅ enthält, wobei Me^{V}₂O₅ ausgewählt ist aus V₂O₅, Ta₂O₅ und/oder Nb₂O₅.

Besonders bevorzugt enthält die Glaskeramik mindestens eines und insbesondere alle der folgenden weiteren Oxide fünfwertiger Elemente Me^{V}₂O₅ in den angegebenen Mengen:

| Komponente | Gew.-% |
|---|---|
| V₂O₅ | 0 bis 5,0, insbesondere 0 bis 4,0 |
| Ta₂O₅ | 0 bis 5,0, insbesondere 0 bis 4,0 |
| Nb₂O₅ | 0 bis 6,0, insbesondere 0 bis 5,0. |

Auch ist eine Glaskeramik bevorzugt, die 0 bis 6,0 Gew.-% Oxid sechswertiger Elemente Me^{VI}O₃ enthält, wobei Me^{VI}O₃ ausgewählt ist aus WO₃ und/oder MoO₃.

Besonders bevorzugt enthält die Glaskeramik mindestens eines und insbesondere alle der folgenden Oxide Me^{VI}O₃ in den angegebenen Mengen:

| Komponente | Gew.-% |
|---|---|
| WO₃ | 0 bis 6,0, insbesondere 0 bis 5,0 |
| MoO₃ | 0 bis 4,0, insbesondere 0 bis 3,0. |

Besonders bevorzugt ist eine Glaskeramik, die mindestens eine und bevorzugt alle der folgenden Komponenten in den angegebenen Mengen enthält:

| Komponente | Gew.-% |
|---|---|
| SiO₂ | 56,0 bis 74,0 |
| Li₂O | 10,0 bis 18,0 |
| CaO | 4,0 bis 17,0 |
| Al₂O₃ | 0, 5 bis 6,0 |
| K₂O | 0 bis 5, 0 |
| P₂O₅ | 1,0 bis 7,0 |
| Me^{I}₂O | 0 bis 13,0 |
| Me^{II}O | 0 bis 6,0 |
| Me^{III}₂O₃ | 0 bis 6,0 |
| Me^{IV}O₂ | 0 bis 8,0 |
| Me^{V}₂O₅ | 0 bis 6,0 |
| Me^{VI}O₃ | 0 bis 6,0, |

wobei Me^{I}₂O, Me^{II}O, Me^{III}₂O₃, Me^{IV}O₂, Me^{V}₂O₅ und Me^{VI}O₃ die oben angegebene Bedeutung haben.

Manche der vorstehend genannten Komponenten können als Färbemittel und/oder Fluoreszenzmittel dienen. Die erfindungsgemäße Glaskeramik kann darüber hinaus noch weitere Färbemittel und/oder Fluoreszenzmittel enthalten, die insbesondere aus anorganischen Pigmenten und/oder Oxiden von d- und f-Elementen, wie z.B. den Oxiden von Sc, Mn, Fe, Co, Pr, Nd, Tb, Dy, Gd, Eu und Yb, ausgewählt sein können. Als weitere Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden.

Die Eigenschaften der Glaskeramik werden maßgeblich durch die Kristallphasen beeinflusst. Die erfindungsgemäße Glaskeramik enthält Lithiumsilikat als eine Kristallphase. Der Begriff "Lithiumsilikat" bezeichnet mindestens eine Kristallphase ausgewählt aus Lithiumdisilikat und Lithiummetasilikat. Mithin enthält die erfindungsgemäße Glaskeramik Lithiumdisilikat, Lithiummetasilikat oder eine Mischung von Lithiumdisilikat und Lithiummetasilikat als Kristallphase.

In einer bevorzugten Ausführungsform enthält die erfindungsgemäße Glaskeramik Lithiummetasilikat oder Lithiumdisilikat als Hauptkristallphase und insbesondere Lithiumdisilikat als Hauptkristallphase.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Die erfindungsgemäße Glaskeramik enthält zusätzlich zu Lithiumsilikat als Kristallphase noch Wollastonit, CaSiO₃, als weitere Kristallphase.

Die erfindungsgemäße Glaskeramik kann ferner weitere Kristallphasen, wie beispielsweise Li₃PO₄, SiO₂, LiAlSi₂O₆, CsAlSi₅O₁₂, Scheelit, LiₓAlₓSi₁₋ₓO₂ und/oder LiAlSi₃O₈ enthalten. Es ist bevorzugt, dass die Glaskeramik Li₃PO₄ als weitere Kristallphase enthält.

Die Art und die Menge der gebildeten Kristallphasen können insbesondere durch die Zusammensetzung des Ausgangsglases sowie das Verfahren zur Herstellung der Glaskeramik gesteuert werden. Die Beispiele veranschaulichen dies anhand der Variation der Zusammensetzung und der Herstellungsverfahren.

Es wurde überraschenderweise gefunden, dass eine Glaskeramik bereitgestellt werden kann, die neben einer Wollastonit-Kristallphase auch eine Lithiumsilikat-Kristallphase aufweist. Dabei war es insbesondere nicht vorhersehbar, dass eine solche Glaskeramik in dem oben beschriebenen bevorzugten Zusammensetzungsbereich gebildet werden kann. Es wurde gefunden, dass die Keimbildung und das Wachstum beider Kristallphasen offenbar nebeneinander in dem Ausgangsglas ablaufen. Dabei waren im Volumen des Ausgangsglases Lithiumsilikat-Kristalle, hingegen an der Oberfläche des Ausgangsglases Wollastonit-Kristalle feststellbar. Danach scheint im Volumen des Ausgangsglases Keimbildung und Wachstum von Lithiumsilikat-Kristallen und demgegenüber an der Oberfläche des Ausgangsglases Keimbildung und Wachstum von Wollastonit-Kristallen aufzutreten. Eine Kristallisation im Volumen eines Glases wird in der Fachwelt auch als Volumenkristallisation und eine Kristallisation an der Oberfläche wird auch als Oberflächenkristallisation bezeichnet.

Die Keimbildung und Kristallisation an der Oberfläche findet bei der Herstellung der erfindungsgemäßen Glaskeramik jedoch nicht ohne weiteres statt. Vielmehr wurde gefunden, dass es notwendig ist, die Oberfläche des Ausgangsglases zu aktivieren, indem es gemahlen wird. Durch diese spezielle Aktivierung gelingt eine reproduzierbare Oberflächenkristallisation von Wollastonit. Dabei kann die Art und Weise des Mahlens, zum Beispiel die Verwendung von unterschiedlichen Mühlen, Einfluss auf die Menge an schließlich kristallisiertem Wollastonit haben.

Die Wollastonit-Menge in der erfindungsgemäßen Glaskeramik wird damit nicht nur durch zum Beispiel den Gehalt an CaO und SiO₂ im Ausgangsglas oder dessen Wärmebehandlung, sondern auch durch die Art und Weise der Aktivierung infolge Mahlens des Ausgangsglases bestimmt.

Des Weiteren wurde gefunden, dass sowohl die Menge an ausgeschiedenem Wollastonit als auch die Größe der Wollastonit-Kristalle einen Einfluss auf die Transluzenz der erfindungsgemäßen Glaskeramik haben. Durch einen hohen Wollastonit-Gehalt oder eine Kristallitgröße von mehr als 10 µm können stark getrübte Glaskeramiken mit einem Kontrastwert (CR-Wert gemäß British Standard BS 5612) von mehr als 90 erzeugt werden. Diese Glaskeramiken eignen sich insbesondere zur Herstellung einer dentalen Abutmentstruktur oder einer dentalen Suprastruktur, auf die eine geeignete Verblendung aufgebracht wird.

Hingegen können bei einem geringen Wollastonit-Gehalt oder einer Kristallitgröße von 5 bis 10 µm transluzente Glaskeramiken mit einem CR-Wert von weniger als 75 erzeugt. Diese Glaskeramiken eignen sich insbesondere zur Herstellung von optisch anspruchsvollen Dentalrestaurationen, wie Kronen, Verblendungen und Inlays.

Die erfindungsgemäße Glaskeramik zeichnet sich weiter dadurch aus, dass sie sogar nach abschließender Ausbildung der der Glaskeramik eine hohe Festigkeit verleihenden Lithiumdisilikat-Kristallphase gut maschinell bearbeitbar ist, um sie z.B. in die Form einer Dentalrestauration zu bringen. Dies ist ein besonderer Vorteil gegenüber konventionellen Lithiumdisilikat-Glaskeramiken, bei denen häufig eine maschinell einfacher bearbeitbare Vorstufe verwendet wird und diese Vorstufe nach der maschinellen Bearbeitung noch einer Wärmebehandlung zu Bildung der gewünschten Lithiumdisilikat-Glaskeramik unterzogen werden muss.

Die erfindungsgemäße Glaskeramik zeichnet sich auch durch eine sehr gute chemische Beständigkeit aus. Zur Bestimmung der chemischen Beständigkeit wurde die Glaskeramik gemäß ISO-Norm 6872 (2008) geprüft, indem der Masseverlust bei Lagerung in wässriger Essigsäure bestimmt wurde. Die erfindungsgemäße Glaskeramik zeigte dabei einen Masseverlust von vorzugsweise weniger als 100 µg/cm².

Die erfindungsgemäße Glaskeramik weist zudem eine biaxiale Bruchfestigkeit σ_{B} von vorzugsweise mindestens 200 MPa und besonders bevorzugt 250 bis 350 MPa auf. Die biaxiale Bruchfestigkeit wurde gemäß ISO 6872 (2008) (Kolben-auf-drei-Kugeln-Prüfung) bestimmt.

Mithin bietet die erfindungsgemäße Glaskeramik eine wünschenswerte Kombination von vorteilhaften optischen und mechanischen Eigenschaften, wie sie insbesondere für ein Dentalmaterial angestrebt wird.

Die Erfindung betrifft ebenfalls Vorstufen mit entsprechender Zusammensetzung, aus denen die erfindungsgemäße Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten als Oxide berechnet werden, so wie es bei Gläsern und Glaskeramiken üblich ist.

Die Erfindung betrifft daher ebenfalls ein Ausgangsglas, das die Komponenten der erfindungsgemäßen Lithiumsilikat-Wollastonit-Glaskeramik enthält. Es sind alle solche Ausführungsformen für die Komponenten des Ausgangsglases bevorzugt, die auch für die Komponenten der erfindungsgemäßen Lithiumsilikat-Wollastonit-Glaskeramik als bevorzugt angegeben sind.

Besonders bevorzugt liegt das Ausgangsglas in gemahlener Form oder in Form eines aus gemahlenem Ausgangsglas gepressten Pulverpresslings vor. In diesen beiden Formen hat das Ausgangsglas durch das Mahlen eine Aktivierung erfahren, die für die spätere Kristallisation von Wollastonit erforderlich ist.

Die Erfindung betrifft weiter auch ein Ausgangsglas, das Keime für die Kristallisation von Lithiumsilikat und/oder Wollastonit enthält.

Ferner betrifft die Erfindung ein Verfahren zur Herstellung der erfindungsgemäßen Lithiumsilikat-Wollastonit-Glaskeramik, bei dem
(a) Ausgangsglas gemahlen wird,
(b) gegebenenfalls das gemahlene Ausgangsglas zu einem Pulverpressling verpresst wird und
(c) das gemahlene Ausgangsglas oder der Pulverpressling mindestens einer Wärmebehandlung bei einer Temperatur im Bereich von 700° bis 950°C für eine Dauer von insbesondere 5 bis 120 min unterzogen wird.

In der Stufe (a) wird das erfindungsgemäße Ausgangsglas gemahlen, um es für die Kristallisation von Wollastonit zu aktivieren.

Das Mahlen erfolgt insbesondere in Mühlen und bevorzugt in Kugelmühlen, Strahlmühlen, wie Gegenstrahlmühlen, oder Schwingmühlen. Die nach dem Mahlen erhaltenen Glasteilchen haben üblicherweise eine mittlere Teilchengröße im Bereich von 10 bis 30 µm, bezogen auf die Anzahl der Teilchen.

Durch die Verwendung unterschiedlicher Mahlverfahren, z.B. durch Einsatz unterschiedlicher Mühlen, kann ein unterschiedlicher Grad an Aktivierung des Ausgangsglases erzielt und damit auch die Menge an schließlich kristallisiertem Wollastonit gesteuert werden.

Das dem Mahlvorgang unterworfene Ausgangsglas liegt vorzugsweise in Form eines Granulates vor. Dabei wird mit dem Begriff "Granulat" ein teilchenförmiges Ausgangsglas bezeichnet. Zur Erzeugung von teilchenförmigem Ausgangsglas kann eine Schmelze des Ausgangsglases in Wasser eingegossen und damit abgeschreckt werden. Dieser Vorgang wird auch als Fritten und das erhaltene Glasgranulat als Glasfritte bezeichnet. Ein Granulat kann aber auch auf andere Weise, wie zum Beispiel durch Abschrecken in einem Walzenstuhl und anschließende Zerkleinerung erzeugt werden.

Die Herstellung des Ausgangsglases erfolgt insbesondere in der Weise, dass eine Mischung von geeigneten Ausgangsmaterialien, wie z.B. Carbonaten, Oxiden und Phosphaten, bei Temperaturen von insbesondere 1300 bis 1700°C, bevorzugt bei etwa 1500 °C, für eine Dauer von 0,5 bis 5 h erschmolzen wird.

In der optionalen Stufe (b) wird das gemahlene Ausgangsglas zu einem Pulverpressling verpresst. Es ist bevorzugt, dass in dem erfindungsgemäßen Verfahren diese Stufe durchgeführt wird.

Im Gegensatz zu einem Glasmonolithen, wie er z.B. durch Gießen einer Glasschmelze in eine Form erhalten wird, zeichnet sich der erfindungsgemäße Pulverpressling durch eine hohe innere Oberfläche aus, an der Kristallisation von Wollastonit erfolgen kann.

Der Pulverpressling kann eine beliebige Geometrie aufweisen. Üblicherweise hat der Pulverpressling bereits im Wesentlichen die Form, die für einen Rohling aus der später erzeugten erfindungsgemäßen Glaskeramik vorgesehen ist.

In der Stufe (c) wird das gemahlene Glas oder der Pulverpressling mindestens einer Wärmebehandlung unterworfen. Diese mindestens eine Wärmebehandlung erfolgt bei einer Temperatur im Bereich von 700° bis 950°C, vorzugsweise 750° bis 900°C, für eine Dauer von insbesondere 5 bis 120 min, vorzugsweise 5 bis 90 min.

Die Wärmebehandlung wird durchgeführt, bis die gewünschte Menge an Lithiumsilikat und Wollastonit kristallisiert ist und damit die erfindungsgemäße Lithiumsilikat-Wollastonit-Glaskeramik gebildet worden ist. Die Wärmebehandlung kann auch stufenweise erfolgen, wobei durch eine erste Wärmebehandlung zunächst eine Vorstufe, wie keimgebildetes Ausgangsglas, und dann durch eine zweite Wärmebehandlung bei einer höheren Temperatur die erfindungsgemäße Glaskeramik gebildet wird. Die Bildung von Keimen für die Kristallisation von Lithiumsilikat findet dabei üblicherweise bei einer Temperatur im Bereich von 460 bis 500°C statt.

Es ist weiter bevorzugt, die Wärmebehandlung so zu wählen, dass es auch zu einem wenigstens teilweisen Sintern, d.h. einem Vorsintern, des gemahlenen Ausgangsglases oder des Pulverpresslings kommt. Besonders bevorzugt ist es, wenn die Wärmebehandlung auch zu einem möglichst vollständigen Sintern, d.h. einem Dichtsintern des gemahlenen Ausgangsglases oder des Pulverpresslings führt.

Aus gemahlenem Ausgangsglas erzeugte dichtgesinterte Glaskeramiken finden dabei vor allem als Beschichtungen auf Substraten, wie dentalen Suprastrukturen, Anwendung. Aus Pulverpresslingen erzeugte dichtgesinterte Glaskeramiken werden vor allem als Rohlinge eingesetzt, aus denen durch geeignete Formgebungsverfahren, wie Pressen und insbesondere maschinelle Bearbeitung, Dentalrestaurationen, wie Brücken, Kronen, Inlays oder Onlays, hergestellt werden können.

Nach Abschluss von Stufe (c) liegt die erfindungsgemäße Lithiumsilikat-Wollastonit-Glaskeramik vor.

Aus der erfindungsgemäßen Glaskeramik und den erfindungsgemäßen Gläsern können dentale Restaurationen, wie Brücken, Inlays, Onlays, Kronen, Veneers, Schalen oder Abutments, hergestellt werden. Die Erfindung betrifft daher deren Verwendung als Dentalmaterial und insbesondere deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 10 bis 30 bar durchzuführen. Beim Verpressen wird durch viskoses Fließen des eingesetzten Materials die gewünschte Formänderung erreicht. Dabei können die erfindungsgemäßen Gläser und Glaskeramiken insbesondere in Form von Rohlingen in beliebiger Form und Größe eingesetzt werden. Für das Verpressen wird bevorzugt die erfindungsgemäße Glaskeramik verwendet. Besonders bevorzugt wird die erfindungsgemäße Glaskeramik mit Lithiumsilikat und insbesondere mit Lithiumdisilikat als Hauptkristallphase verwendet.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Dabei können die erfindungsgemäßen Gläser und Glaskeramiken insbesondere in Form von Rohlingen eingesetzt werden. Diese sind von ihrer Form regelmäßig an den Typ der für die maschinelle Bearbeitung eingesetzten Maschine angepasst. Für die maschinelle Bearbeitung wird insbesondere die erfindungsgemäße Glaskeramik verwendet. Besonders bevorzugt wird die erfindungsgemäße Glaskeramik mit Lithiumsilikat und insbesondere mit Lithiumdisilikat als Hauptkristallphase verwendet.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser als Dentalmaterial und insbesondere zur Herstellung dentaler Restaurationen, wie Kronen, Brücken und Abutments.

Die Erfindung betrifft mithin auch ein Verfahren zur Herstellung einer dentalen Restauration, insbesondere Brücke, Inlay, Onlay, Veneer, Abutment, Teilkrone, Krone oder Schale, bei dem der erfindungsgemäßen Glaskeramik oder dem erfindungsgemäßen Glas durch Verpressen oder durch maschinelle Bearbeitung, insbesondere im Rahmen eines CAD/CAM-Verfahrens, die Form der gewünschten dentalen Restauration gegeben wird.

Die Erfindung wird im Folgenden anhand von sie nichtbeschränkenden Beispielen näher erläutert.

### Beispiele

### Beispiele 1 bis 32 - Zusammensetzung und Kristallphasen

Es wurden insgesamt 32 Gläser und Glaskeramiken mit der in Tabelle I angegebenen Zusammensetzung hergestellt.

Dabei bedeuten in Tabelle I:
- T_{g}: Glasübergangstemperatur, bestimmt mittels DSC
- T_{S} und t_{S}: Angewendete Temperatur und Zeit für Erschmelzung des Ausgangsglases
- T_{Kb} und t_{Kb}: Angewendete Temperatur und Zeit für die Keimbildung des Ausgangsglases
- T_{Sinter} und t_{Sinter}: Angewendete Temperatur und Zeit für die Wärmebehandlung zur Kristallisation und Sinterung von Presslingen
- T_{Press} und t_{Press}: Angewendete Temperatur und Haltezeit bei der Temperatur für das Verpressen von kristallisierten Presslingen
- CR-Wert: Kontrastwert der Glaskeramik gemäß British Standard BS 5612
- Li₂Si₂O₅: Lithiumdisilikat
- Li₂SiO₃: Lithiummetasilikat
- CaSiO₃: Wollastonit
- KM: Mit Kugelmühle gemahlen
- AFG: Mit Strahlmühle gemahlen

In den Beispielen 1 bis 32 wurden Gläser aus üblichen Rohstoffen in einem Platintiegel bei der Temperatur T_{S} für eine Dauer t_{S} erschmolzen. Durch Eingießen der erschmolzenen Ausgangsgläser in Wasser wurden Glasfritten, d.h. Glasgranulate, hergestellt. Für die Weiterverarbeitung der Glasfritten zu erfindungsgemäßen Glaskeramiken wurden die im Folgenden angegebenen drei Verfahrensvarianten A), B) und C) benutzt.

Es zeigte sich, dass in Abhängigkeit von dem P₂O₅-Gehalt die erhaltenen Lithiumdisilikat-Kristalle eine Grösse von etwa 500 nm bis 6 µm hatten. Dabei bildeten die Lithiumdisilikat-Kristalle ein vernetztes und verzahntes Gefüge aus, was vermutlich auch für die guten mechanischen Eigenschaften der Glaskeramiken verantwortlich ist. Die Wollastonit-Kristalle lagen vereinzelt im Lithiumdisilikat-Gefüge vor und sie besaßen eine Größe von etwa 5 µm bis mehr als 10 µm.

### A) Schwingmühlen

Die gemäß den Beispielen 1 bis 30 hergestellten Glasfritten wurden mit einer Schwingmühle KM100 der Firma Retsch GmbH, Haan, Deutschland, oder einer Zirkonoxidschwingmühle RM31 der Firma Retsch GmbH, Haan, Deutschland auf eine mittlere Korngröße von <90 µm, bezogen auf die Anzahl der Teilchen, gemahlen. Das gemahlene Glaspulver wurde anschließend uniaxial zu einem kleinen Zylinder verpresst und in einem Ofen vom Typ Programat (Ivoclar Vivadent AG) bei der Temperatur T_{Sinter} für die Dauer t_{Sinter} kristallisiert und gesintert. An den hergestellten Prüfkörpern wurden Röntgenbeugungsanalysen zur Bestimmung der vorhandenen Kristallphasen sowie Farbmessungen durchgeführt.

### B) Strahlmühle

Die Glasfritte mit der Zusammensetzung gemäß Beispiel 31 wurde in einer Gegenstrahlmühle AFG 100, der Firma Hosokawa Alpine, auf eine mittlere Korngröße von 23 µm, bezogen auf die Anzahl der Teilchen, gemahlen. Das gemahlene Glaspulver wurde daraufhin uniaxial verpresst und in einem Ofen vom Typ Programat (Ivoclar Vivadent AG) bei der Temperatur T_{Sinter} für die Dauer t_{Sinter} kristallisierte und gesintert. An den so hergestellten Prüfkörpern wurden Röntgenbeugungsanalysen durchgeführt.

### C) Kugelmühle

Die Glasfritten mit der Zusammensetzung gemäß Beispiel 31 und 32 wurden in einer Kugelmühle auf eine mittlere Korngröße von 23 µm, bezogen auf die Anzahl der Teilchen, gemahlen. Die Kugelmühle hatte als Mahlraum einen zylinderförmigen Porzellanbehälter mit einem Fassungsvermögen von 5 l. Als Mahlkörper wurde die folgende Mischung aus Porzellanmahlkugeln verwendet: 0,9 kg mit Durchmesser 10 mm, 1,8 kg mit Durchmesser 20 mm und 0,9 kg mit Durchmesser 30 mm. Die gemahlenen Glaspulver wurden dann uniaxial verpresst und in einem Ofen vom Typ Programat (Ivoclar Vivadent AG) bei der Temperatur T_{Sinter} für die Dauer t_{Sinter} kristallisiert und gesintert. An den so hergestellten Prüfkörpern wurden Röntgenbeugungsanalysen zur Bestimmung der Kristallphasen durchgeführt. Der Gehalt an Wollastonit-Kristallen war bei diesen Glaskeramiken höher als bei den nach den Varianten A) und B) hergestellten Glaskeramiken.

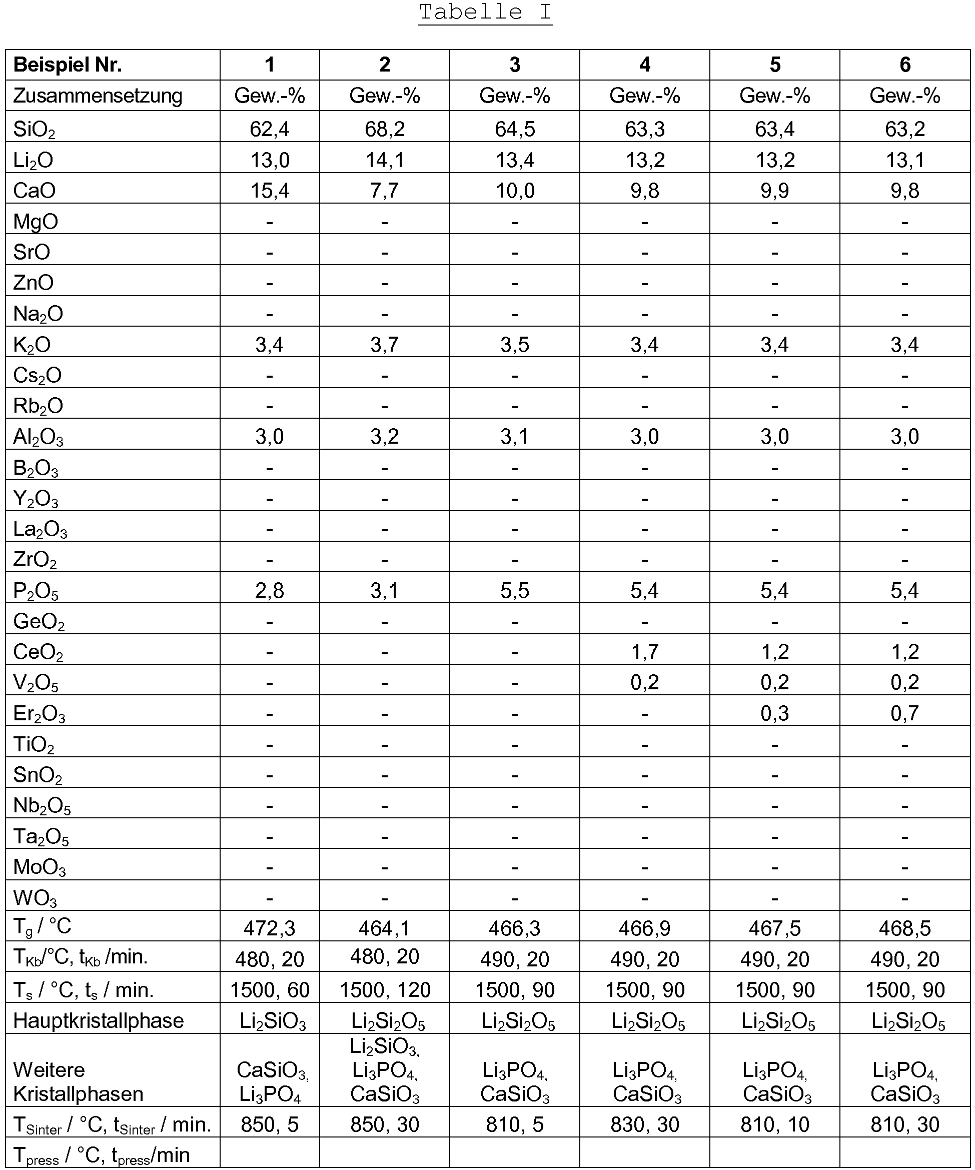

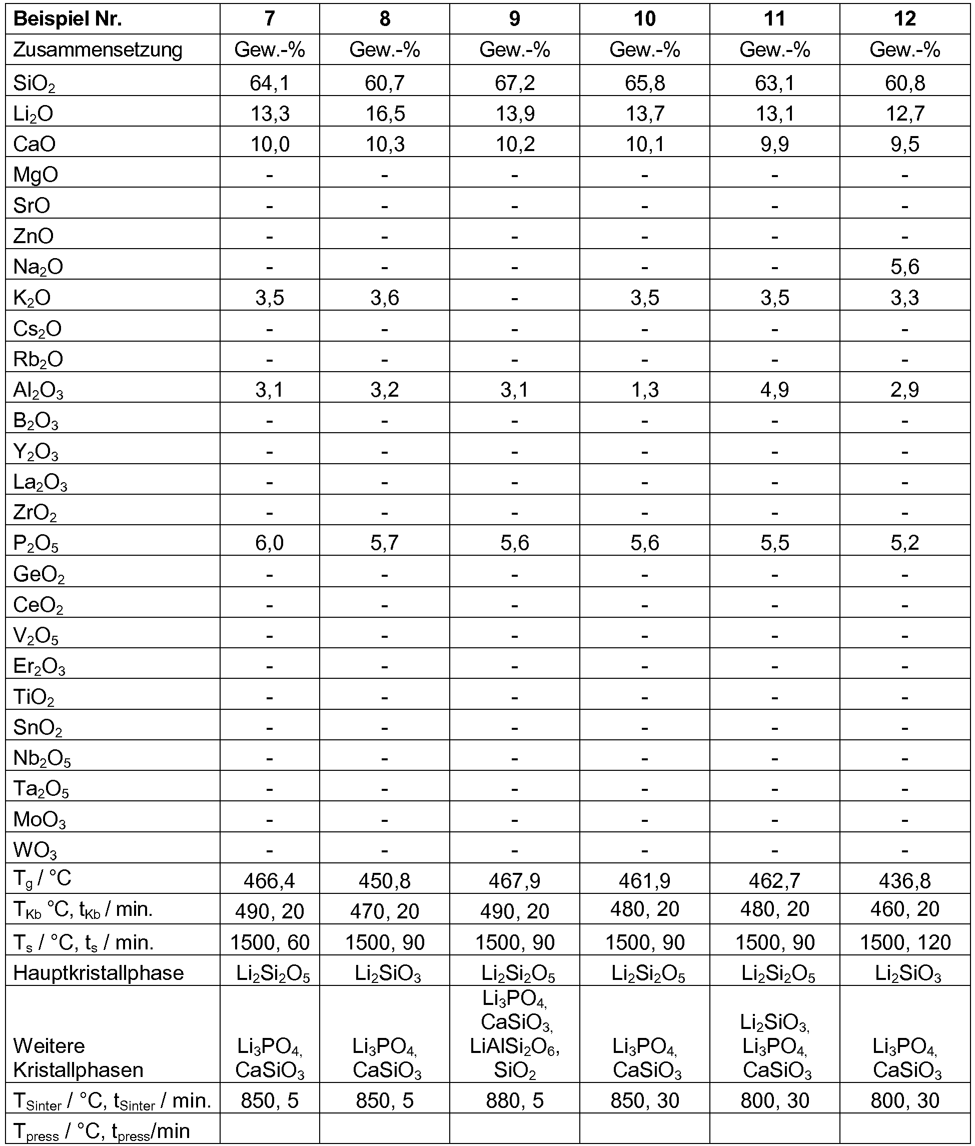

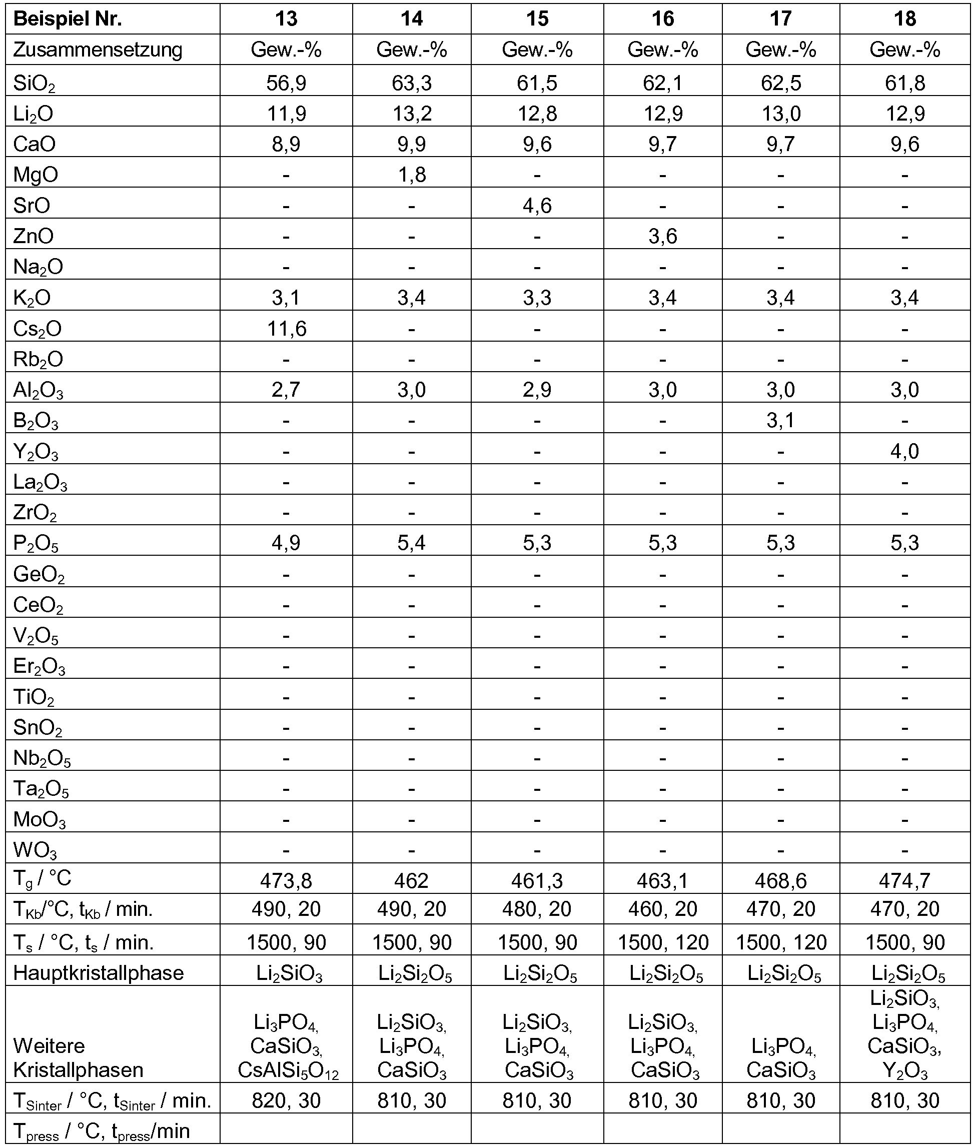

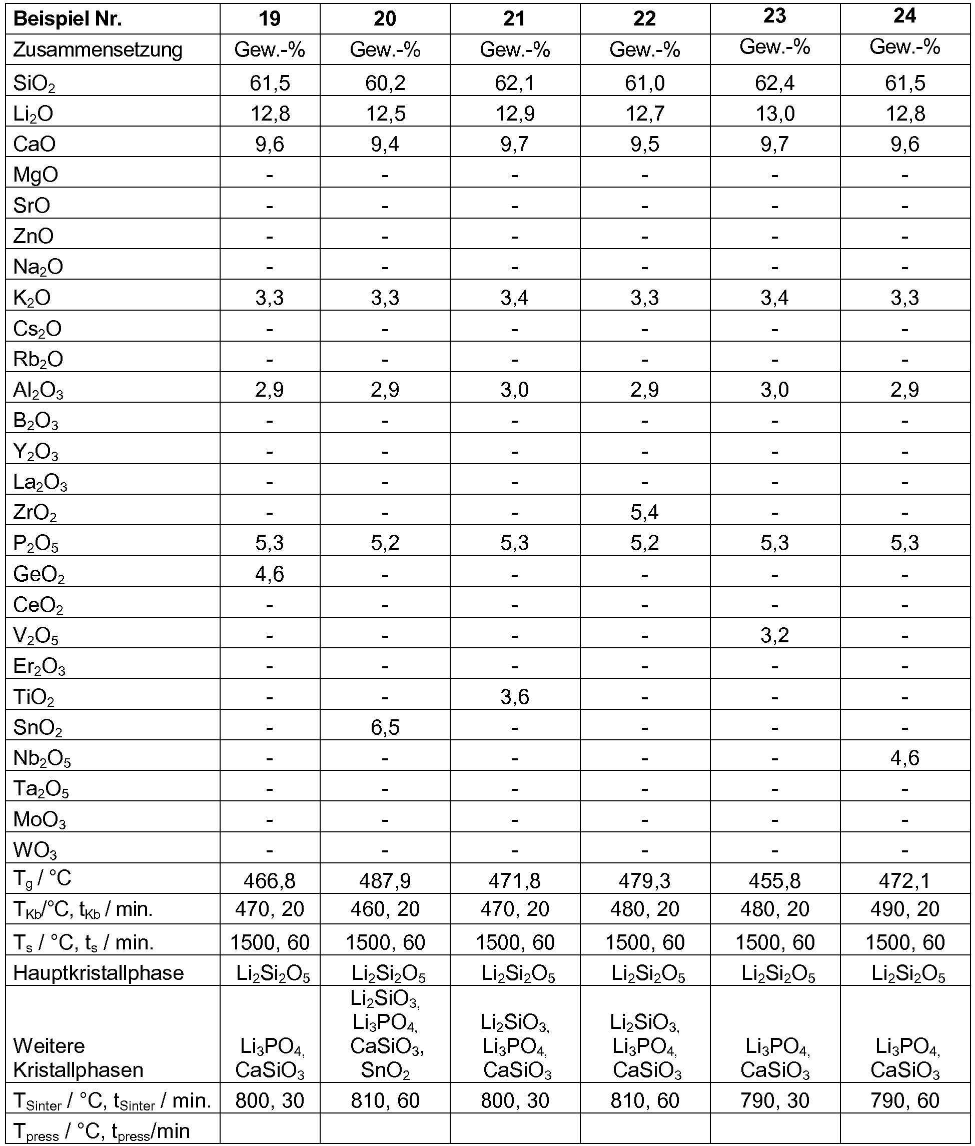

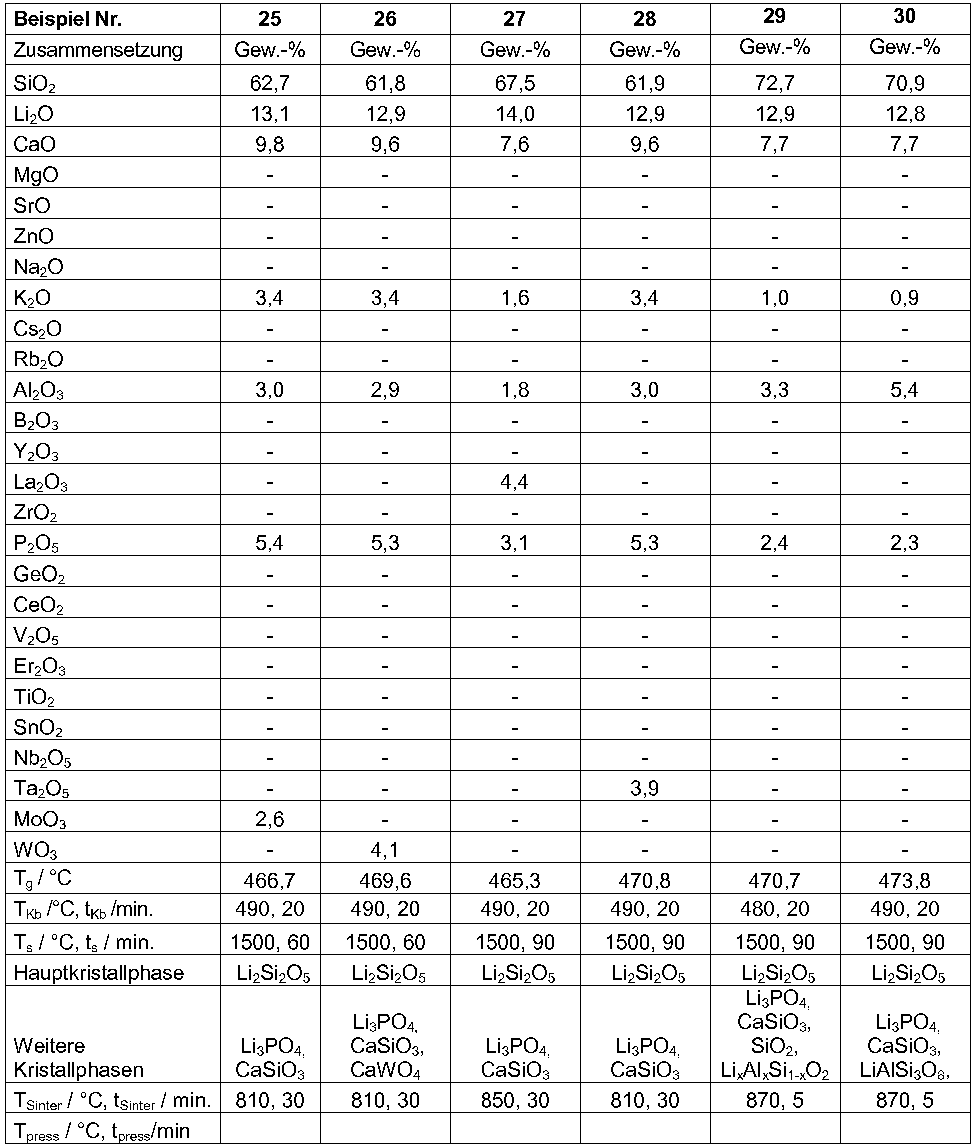

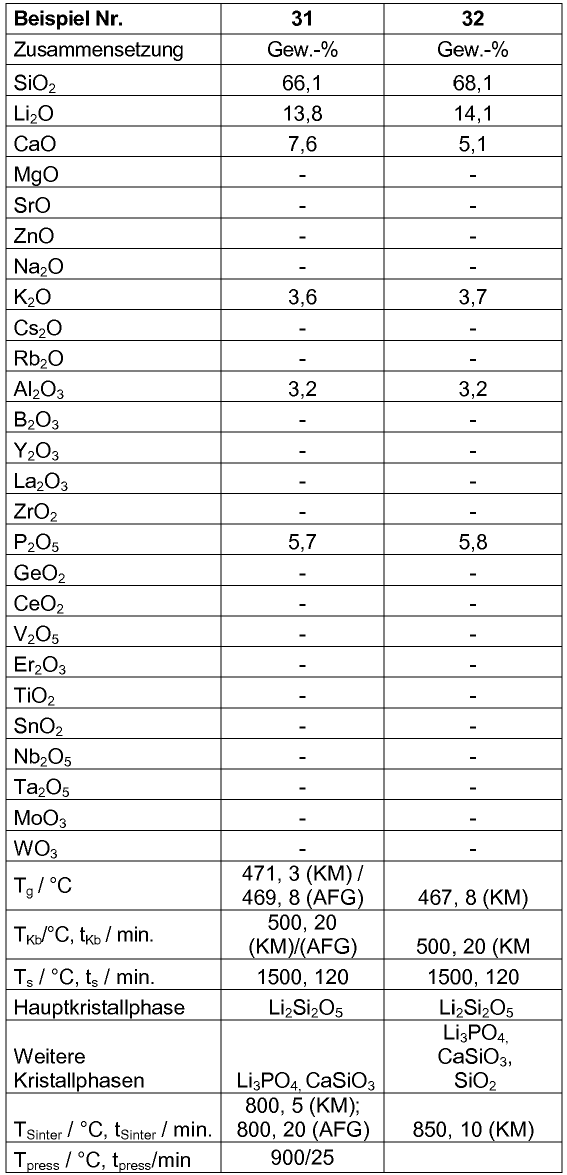

### Beispiel 33 - Heißpressen

Ein Glas mit der Zusammensetzung gemäß Beispiel 31 wurde in einem Platintiegel bei einer Temperatur von 1500°C erschmolzen und anschließend in Wasser gegossen. Die so hergestellte Glasfritte wurde mit einer Gegenstrahlmühle AFG 100, der Firma Hosokawa Alpine, auf eine mittlere Korngröße von 23 µm, bezogen auf die Anzahl der Teilchen, gemahlen. Aus dem erhaltenen Glaspulver wurde durch uniaxiales Pressen ein Pulverpressling hergestellt. Der Pressrohling wurde bei einer Temperatur von 800°C und einer Haltezeit von 20 min in einem Ofen vom Typ Programat dichtgesintert. Der dichtgesinterte und dabei bereits kristallisierte Rohling wurde anschließend durch Heißpressen bei einer Temperatur von 900°C bei einer Haltezeit von 25 min verpresst. Der verpresste Probekörper hatte einen CR-Wert von 86,65 und einen thermischen Ausdehnungskoeffizienten von 10.75 * 10⁻⁶ K⁻¹, gemessen im Bereich von 100 bis 500°C.

### Beispiel 34 - Mechanische Bearbeitbarkeit

Zum Test der mechanischen Bearbeitbarkeit wurden Glaspulver gemäß den Beispielen 3, 5 und 31 uniaxial zu Blöcken verpresst und in einem Ofen vom Typ Programat dichtgesintert. Auf die so hergestellten Glaskeramikblöcke wurden daraufhin entsprechende Halter aufgeklebt, und sie wurden mit einer CAD/CAM-Schleifeinheit (Sirona InLab) bearbeitet. Zum Test der Bearbeitbarkeit wurden dabei Biaxialprüfkörper aus den Blöcken geschliffen, was ohne Probleme und nur mit geringem Werkzeugverschleiß möglich war.

## Patentansprüche

1. Lithiumsilikat-Wollastonit-Glaskeramik, die Lithiumsilikat als eine Kristallphase und Wollastonit als eine weitere Kristallphase enthält, wobei die Glaskeramik 0,5 bis 6,0 Gew.-% Al₂O₃ und 1,0 bis 7,0 Gew.-% P₂O₅ enthält.

2. Glaskeramik nach Anspruch 1, die 55,0 bis 74,0, insbesondere 56,0 bis 73,0 und bevorzugt 60,0 bis 69,0 Gew.-% SiO₂ enthält.

3. Glaskeramik nach Anspruch 1 oder 2, die 10,0 bis 18,0, insbesondere 11,0 bis 17,0 und bevorzugt 12,0 bis 16,5 Gew.-% Li₂O enthält.

4. Glaskeramik nach einem der Ansprüche 1 bis 3, die 4,0 bis 17,0, insbesondere 5,0 bis 16,0 und bevorzugt 7,0 bis 15,0 Gew.-% CaO enthält.

5. Glaskeramik nach einem der Ansprüche 1 bis 4, die 0,5 bis 5,0 und bevorzugt 1,0 bis 4,0 Gew.-% Al₂O₃ enthält.

6. Glaskeramik nach einem der Ansprüche 1 bis 5, die 0 bis 5,0, insbesondere 0 bis 4,5 und bevorzugt 0,5 bis 4,0 Gew.-% K₂O enthält.

7. Glaskeramik nach einem der Ansprüche 1 bis 6, die 2,0 bis 6,0 und bevorzugt 3,0 bis 6,0 Gew.-% P₂O₅ enthält.

8. Glaskeramik nach einem der Ansprüche 1 bis 7 die 0 bis 13,0, bevorzugt 0 bis 12,0 und besonders bevorzugt 0 bis 11,0 Gew.-% weiteres Alkalimetalloxid Me^{I}₂O enthält, wobei Me^{I}₂O ausgewählt ist aus Na₂O, Rb₂O und/oder Cs₂O.

9. Glaskeramik nach einem der Ansprüche 1 bis 8, die 0 bis 6,0 und bevorzugt 0 bis 5,0 Gew.-% weiteres Oxid zweiwertiger Elemente Me^{II}O enthält, wobei Me^{II}O ausgewählt ist aus MgO, SrO und/oder ZnO.

10. Glaskeramik nach einem der Ansprüche 1 bis 9, die 0 bis 6,0 und bevorzugt 0 bis 5,0 Gew.-% Oxid dreiwertiger Elemente Me^{III}₂O₃ enthält, wobei Me^{III}₂O₃ ausgewählt ist aus B₂O₃, Y₂O₃, La₂O₃ und/oder Er₂O₃.

11. Glaskeramik nach einem der Ansprüche 1 bis 10, die 0 bis 8,0 und bevorzugt 0 bis 7,0 Gew.-% weiteres Oxid vierwertiger Elemente Me^{IV}O₂ enthält, wobei Me^{IV}O₂ ausgewählt ist aus ZrO₂, GeO₂, CeO₂, TiO₂ und/oder SnO₂.

12. Glaskeramik nach einem der Ansprüche 1 bis 11, die 0 bis 6,0 und bevorzugt 0 bis 5,0 Gew.-% weiteres Oxid fünfwertiger Elemente Me^{V}₂O₅ enthält, wobei Me^{V}₂O₅ ausgewählt ist aus V₂O₅, Ta₂O₅ und/oder Nb₂O₅.

13. Glaskeramik nach einem der Ansprüche 1 bis 12, die 0 bis 6,0 Gew.-% Oxid sechswertiger Elemente Me^{VI}O₃ enthält, wobei Me^{VI}O₃ ausgewählt ist aus WO₃ und/oder MoO₃.

14. Glaskeramik nach einem der Ansprüche 1 bis 13, die mindestens eine und bevorzugt alle folgenden Komponenten in den angegebenen Mengen enthält:
| Komponente | Gew.-% |
|---|---|
| SiO₂ | 56,0 bis 74,0 |
| Li₂O | 10,0 bis 18,0 |
| CaO | 4,0 bis 17,0 |
| Al₂O₃ | 0,5 bis 6,0 |
| K₂O | 0 bis 5,0 |
| P₂O₅ | 1,0 bis 7,0 |
| Me^{I}₂O | 0 bis 13,0 |
| Me^{II}O | 0 bis 6,0 |
| Me^{III}₂O₃ | 0 bis 6,0 |
| Me^{IV}O₂ | 0 bis 8,0 |
| Me^{V}₂O₅ | 0 bis 6,0 |
| Me^{VI}O₃ | 0 bis 6,0. |

15. Glaskeramik nach einem der Ansprüche 1 bis 14, die Lithiumdisilikat und/oder Lithiummetasilikat enthält.

16. Glaskeramik nach einem der Ansprüche 1 bis 15, die Lithiummetasilikat oder Lithiumdisilikat und insbesondere Lithiumdisilikat als Hauptkristallphase enthält.

17. Glaskeramik nach einem der Ansprüche 1 bis 16, die Lithiumphosphat als weitere Kristallphase enthält.

18. Glaskeramik nach einem der Ansprüche 1 bis 17, die in Form von einem Rohling oder einer dentalen Restauration vorliegt.

19. Ausgangsglas, das die Komponenten der Glaskeramik nach einem der Ansprüche 1 bis 14 enthält und in Form von einem gemahlenen Pulver oder einem Pressling aus gemahlenem Pulver vorliegt und insbesondere Keime für die Kristallisation von Lithiumsilikat und/oder Wollastonit enthält.

20. Verfahren zu Herstellung der Glaskeramik gemäß einem der Ansprüche 1 bis 18, bei dem
(a) ein Ausgangsglas, das die Komponenten der Glaskeramik nach einem der Ansprüche 1 bis 14 und insbesondere Keime für die Kristallisation von Lithiumsilikat und/oder Wollastonit enthält, gemahlen wird,
(b) gegebenenfalls das gemahlene Ausgangsglas zu einem Pulverpressling verpresst wird und
(c) das gemahlene Ausgangsglas oder der Pulverpressling mindestens einer Wärmebehandlung bei einer Temperatur im Bereich von 700°C bis 950°C für eine Dauer von insbesondere 5 bis 120 min unterzogen wird.

21. Verwendung des Ausgangsglases gemäß Anspruch 19 als Dentalmaterial, insbesondere zur Herstellung dentaler Restaurationen.

22. Verwendung einer Glaskeramik, die Lithiumsilikat als eine Kristallphase und Wollastonit als eine weitere Kristallphase enthält, als Dentalmaterial, insbesondere zur Herstellung dentaler Restaurationen.

23. Verwendung nach Anspruch 22, wobei die Glaskeramik die Glaskeramik gemäß einem der Ansprüche 1 bis 18 ist.

24. Verwendung zur Herstellung dentaler Restaurationen nach Anspruch 22 oder 23, wobei der Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

## Claims

1. Lithium silicate-wollastonite glass ceramic, which comprises lithium silicate as a crystal phase and wollastonite as a further crystal phase, wherein the glass ceramic comprises 0.5 to 6.0 wt.-% Al₂O₃ and 1.0 to 7.0 wt.-% P₂O₅.

2. Glass ceramic according to claim 1, which comprises 55.0 to 74.0, in particular 56.0 to 73.0 and preferably 60.0 to 69.0 wt.-% SiO₂.

3. Glass ceramic according to claim 1 or 2, which comprises 10.0 to 18.0, in particular 11.0 to 17.0 and preferably 12.0 to 16.5 wt.-% LiO₂.

4. Glass ceramic according to any one of claims 1 to 3, which comprises 4.0 to 17.0, in particular 5.0 to 16.0 and preferably 7.0 to 15.0 wt.-% CaO.

5. Glass ceramic according to any one of claims 1 to 4, which comprises 0.5 to 5.0 and preferably 1.0 to 4.0 wt.-% Al₂O₃.

6. Glass ceramic according to any one of claims 1 to 5, which comprises 0 to 5.0, in particular 0 to 4.5 and preferably 0.5 to 4.0 wt.-% K₂O.

7. Glass ceramic according to any one of claims 1 to 6, which comprises 2.0 to 6.0 and preferably 3.0 to 6.0 wt.-% P₂O₅.

8. Glass ceramic according to any one of claims 1 to 7, which comprises 0 to 13.0, preferably 0 to 12.0 and particularly preferred 0 to 11.0 wt.-% further alkali metal oxide Me^{I}₂O, wherein Me^{I}₂O is selected from Na₂O, Rb₂O and/or Cs₂O.

9. Glass ceramic according to any one of claims 1 to 8, which comprises 0 to 6.0 and preferably 0 to 5.0 wt.-% further oxide of divalent elements Me^{II}O, wherein Me^{II}O is selected from MgO, SrO and/or ZnO.

10. Glass ceramic according to any one of claims 1 to 9, which comprises 0 to 6.0 and preferably 0 to 5.0 wt.-% oxide of trivalent elements Me^{III}₂O₃, wherein Me^{III}₂O₃ is selected from B₂O₃, Y₂O₃, La₂O₃, and/or Er₂O₃.

11. Glass ceramic according to any one of claims 1 to 10, which comprises 0 to 8.0 and preferably 0 to 7.0 wt.-% further oxide of tetravalent elements Me^{IV}O₂, wherein Me^{IV}O₂ is selected from ZrO₂, GeO₂, CeO₂, TiO₂ and/or SnO₂.

12. Glass ceramic according to any one of claims 1 to 11, which comprises 0 to 6.0 and preferably 0 to 5.0 wt.-% further oxide of pentavalent elements Me^{V}₂O₅, wherein Me^{V}₂O₅ is selected from V₂O₅, Ta₂O₅, and/or Nb₂O₅.

13. Glass ceramic according to any one of claims 1 to 12, which comprises 0 to 6.0 wt.-% oxide of hexavalent elements Me^{VI}O₃, wherein Me^{VI}O₃ is selected from WO₃ and/or MoO₃.

14. Glass ceramic according to any one of claims 1 to 13, which comprises at least one and preferably all of the following components in the indicated amounts:
| Component | Wt.-% |
|---|---|
| SiO₂ | 56.0 to 74.0 |
| Li₂O | 10.0 to 18.0 |
| CaO | 4.0 to 17.0 |
| Al₂O₃ | 0.5 to 6.0 |
| K₂O | 0 to 5.0 |
| P₂O₅ | 1.0 to 7.0 |
| Me^{I}₂O | 0 to 13.0 |
| Me^{II}O | 0 to 6.0 |
| Me^{III}₂O₃ | 0 to 6.0 |
| Me^{IV}O₂ | 0 to 8.0 |
| Me^{V}₂O₅ | 0 to 6.0 |
| Me^{VI}O₃ | 0 to 6.0. |

15. Glass ceramic according to any one of claims 1 to 14, which comprises lithium disilicate and/or lithium metasilicate.

16. Glass ceramic according to any one of claims 1 to 15, which comprises lithium metasilicate or lithium disilicate and in particular lithium disilicate as main crystal phase.

17. Glass ceramic according to any one of claims 1 to 16, which comprises lithium phosphate as further crystal phase.

18. Glass ceramic according to any one of claims 1 to 17, which is present in the form of a blank or a dental restoration.

19. Starting glass, which comprises the components of the glass ceramic according to any one of claims 1 to 14 and which is present in the form of a ground powder or a compact made from ground powder and which comprises in particular nuclei for the crystallization of lithium silicate and/or wollastonite.

20. Process for the preparation of the glass ceramic according to any one of claims 1 to 18, comprising
(a) grinding of a starting glass, which comprises the components of the glass ceramic according to any one of claims 1 to 14 and in particular nuclei for the crystallization of lithium silicate and/or wollastonite,
(b) optionally pressing the ground starting glass to form a powder compact and
(c) subjecting the ground starting glass or the powder compact to at least one heat treatment at a temperature in the range of 700°C to 950°C for a period of in particular 5 to 120 min.

21. Use of a starting glass according to claim 19 as dental material, in particular for the preparation of dental restorations.

22. Use of a glass ceramic, which comprises lithium silicate as a crystal phase and wollastonite as a further crystal phase, as dental material, in particular for the preparation of dental restorations.

23. Use according to claim 22, wherein the glass ceramic is the glass ceramic according to any one of claims 1 to 18.

24. Use for the preparation of dental restorations according to claim 22 or 23, wherein the glass ceramic is given the shape of the desired dental restoration, in particular bridge, inlay, onlay, veneer, abutment, partial crown, crown or facet, by pressing or machining.

## Revendications

1. Vitrocéramique de silicate de lithium et de wollastonite qui contient du silicate de lithium en tant que principale phase cristalline et de la wollastonite en tant qu'autre phase cristalline, laquelle vitrocéramique contient de 0,5 à 6,0 % en poids de Al₂O₃ et de 1,0 à 7,0 % en poids de P₂O₅.

2. Vitrocéramique conforme à la revendication 1, qui contient de 55,0 à 74,0, en particulier de 56,0 à 73,0 et de préférence de 60,0 à 69,0 % en poids de SiO₂.

3. Vitrocéramique conforme à la revendication 1 ou 2, qui contient de 10,0 à 18,0, en particulier de 11,0 à 17,0 et de préférence de 12,0 à 16,5 % en poids de Li₂O.

4. Vitrocéramique conforme à l'une des revendications 1 à 3, qui contient de 4,0 à 17,0, en particulier de 5,0 à 16,0 et de préférence de 7,0 à 15,0 % en poids de CaO.

5. Vitrocéramique conforme à l'une des revendications 1 à 4, qui contient de 0,5 à 5,0 et de préférence de 1,0 à 4,0 % en poids de Al₂O₃.

6. Vitrocéramique conforme à l'une des revendications 1 à 5, qui contient de 0 à 5,0, en particulier de 0 à 4,5 et de préférence de 0,5 à 4,0 % en poids de K₂O.

7. Vitrocéramique conforme à l'une des revendications 1 à 6, qui contient de 2,0 à 6,0 et de préférence de 3,0 à 6,0 % en poids de P₂O₅.

8. Vitrocéramique conforme à l'une des revendications 1 à 7, qui contient de 0 à 13,0, de préférence de 0 à 12,0 et mieux encore de 0 à 11,0 % en poids d'autres oxydes de métaux alcalins Me^{I}₂O, étant entendu que Me^{I}₂O est choisi parmi Na₂O, Rb₂O et/ou Cs₂O.

9. Vitrocéramique conforme à l'une des revendications 1 à 8, qui contient de 0 à 6,0 et de préférence de 0 à 5,0 % en poids d'autres oxydes d'éléments divalents Me^{II}O, étant entendu que Me^{II}O est choisi parmi MgO, SrO et/ou ZnO.

10. Vitrocéramique conforme à l'une des revendications 1 à 9, qui contient de 0 à 6,0 et de préférence de 0 à 5,0 % en poids d'oxydes d'éléments trivalents Me^{III}₂O₃, étant entendu que M^{III}₂O₃ est choisi parmi B₂O₃, Y₂O₃, La₂O₃ et/ou Er₂O₃.

11. Vitrocéramique conforme à l'une des revendications 1 à 10, qui contient de 0 à 8,0 et de préférence de 0 à 7,0 % en poids d'autres oxydes d'éléments tétravalents Me^{IV}O₂, étant entendu que Me^{IV}O₂ est choisi parmi ZrO₂, GeO₂, CeO₂, TiO₂ et/ou SnO₂.

12. Vitrocéramique conforme à l'une des revendications 1 à 11, qui contient de 0 à 6,0 et de préférence de 0 à 5,0 % en poids d'autres oxydes d'éléments pentavalents Me^{V}₂O₅, étant entendu que Me^{V}₂O₅ est choisi parmi V₂O₅, Ta₂O₅ et/ou Nb₂O₅.

13. Vitrocéramique conforme à l'une des revendications 1 à 12, qui contient de 0 à 6,0 % en poids d'oxydes d'éléments hexavalents Me^{VI}O₃, étant entendu que Me^{VI}O₃ est choisi parmi WO₃ et/ou MoO₃.

14. Vitrocéramique conforme à l'une des revendications 1 à 13, qui contient au moins l'un des composants suivants, et de préférence les contient tous, en les proportions indiquées :
| Composant | % en poids |
|---|---|
| SiO₂ | 56,0 - 74,0 |
| Li₂O | 10,0 - 18,0 |
| CaO | 4,0 - 17,0 |
| Al₂O₃ | 0,5 - 6,0 |
| K₂O | 0 - 5,0 |
| P₂O₅ | 1,0 - 7,0 |
| Me^{I}₂O | 0 - 13,0 |
| Me^{II}O | 0 - 6,0 |
| Me^{III}₂O₃ | 0 - 6,0 |
| Me^{IV}O₂ | 0 - 8,0 |
| Me^{V}₂O₅ | 0 - 6,0 |
| Me^{VI}O₃ | 0 - 6,0 |

15. Vitrocéramique conforme à l'une des revendications 1 à 14, qui contient du disilicate de lithium et/ou du métasilicate de lithium.

16. Vitrocéramique conforme à l'une des revendications 1 à 15, qui contient, en tant que principale phase cristalline, du métasilicate de lithium ou du disilicate de lithium, et en particulier, du disilicate de lithium.

17. Vitrocéramique conforme à l'une des revendications 1 à 16, qui contient, en tant qu'autre phase cristalline, du phosphate de lithium.

18. Vitrocéramique conforme à l'une des revendications 1 à 17, qui se présente sous la forme d'une ébauche ou d'une pièce de restauration dentaire.

19. Verre de départ qui contient les constituants d'une vitrocéramique conforme à l'une des revendications 1 à 14, qui se présente sous la forme d'une poudre moulue ou d'un comprimé obtenu à partir d'une poudre moulue, et qui contient en particulier des germes pour la cristallisation du silicate de lithium et/ou de la wollastonite.

20. Procédé de fabrication d'une vitrocéramique conforme à l'une des revendications 1 à 18, dans lequel
a) on moud un verre de départ qui contient les constituants d'une vitrocéramique conforme à l'une des revendications 1 à 14 et qui contient en particulier des germes pour la cristallisation du silicate de lithium et/ou de la wollastonite,
b) en option, on comprime le verre de départ moulu pour en faire un comprimé de poudre,
c) et l'on soumet le verre de départ moulu ou le comprimé de poudre à au moins un traitement thermique, à une température située dans l'intervalle allant de 700 à 950 °C et durant un laps de temps de 5 à 120 minutes.

21. Utilisation d'un verre de départ conforme à la revendication 19 en tant que matériau dentaire, en particulier pour la fabrication de pièces de restauration dentaire.

22. Utilisation d'une vitrocéramique, qui contient du silicate de lithium en tant que phase cristalline et de la wollastonite en tant qu'autre phase cristalline, en tant que matériau dentaire, en particulier pour la fabrication de pièces de restauration dentaire.

23. Utilisation conforme à la revendication 22, dans laquelle la vitrocéramique est conforme à l'une des revendications 1 à 18.

24. Utilisation pour la fabrication de pièces de restauration dentaire, conforme à la revendication 22 ou 23, dans laquelle on donne à la vitrocéramique, par compression ou travail à la machine, la forme des pièces de restauration dentaire voulues, en particulier bridges, incrustations inlay ou onlay, facettes « veneer », piliers, couronnes partielles, couronnes ou plaquettes.
